# EUROPEAN PATENT SPECIFICATION

(11) **EP 0 660 713 B1**
(45) Date of publication and mention of the grant of the patent: **24.10.2001**
(21) Application number: 92900691.4
(22) Date of filing: 12.12.1991
(51) Int. Cl.: A61K 31/41, A61K 31/415, A61P 9/00

(54) **USE OF ANGIOTENSIN II ANTAGONISTS IN THE TREATMENT OF LEFT VENTRICULAR HYPERTROPHY**
VERWENDUNG VON ANGIOTENSIN-II-ANTAGONISTEN IN DER BEHANDLUNG DER LINKEN VENTRIKULÄREN HYPERTROPHIE
UTILISATION D'AGENTS ANTAGONISTES DE L'ANGIOTENSINE II DANS LE TRAITEMENT DE L'HYPERTROPHIE VENTRICULAIRE GAUCHE

(30) Priority: 14.12.1990 GB 9027209
(43) Date of publication of application: 05.07.1995
(73) Proprietor: SMITHKLINE BEECHAM PLC, Brentford, Middlesex TW8 9EP (GB)
(72) Inventor: HILL, James, Brentford, Middlesex TW8 9EP (GB)
(74) Representative: Thompson, Clive Beresford
(86) International application number: GB9102219
(87) International publication number: WO9210181

(56) References cited:
- EP-A- 0 323 841
- EP-A- 0 403 158
- EP-A- 0 403 159
- R. BERKOW et al.: "The Merck manual of Diagnosis and therapy", 15th edition, 1987, Rahway, US, pages 519-522, see paragraph: "Hypertrophic cardiomyopathy", especially page 521

## Description

### FIELD OF THE INVENTION

The present invention relates to therapeutic agents that are angiotensin II (AII) receptor antagonists useful in the treatment of left ventricular hypertrophy.

### BACKGROUND OF THE INVENTION

Interruption of the renin-angiotensin system (RAS) with converting enzyme inhibitors, such as captopril, has proved clinically useful in the treatment of certain disease states, such as hypertension and congestive heart failure [Abrams *et al*, Federation Proc., 43:1314 (1984)]. Furthermore, evidence suggests that inhibition of this system may be beneficial in treating left ventricular hypertrophy. Since AII is the biologically active component of the renin-angiotensin system responsible for the system's peripheral effects, the most direct approach towards inhibition of RAS and in particular AII-induced left ventricular hypertrophy would be blockade of angiotensin II at its receptor.

Carini *et al*, in EP Application 0 323 841, filed January 5, 1989, disclose substituted pyrrole, pyrazole and triazole compounds useful as angiotensin II receptor antagonists in the treatment of hypertension and in the treatment of congestive heart failure.

Heitsch *et al*, in EP Application 0 492 105, filed November 11, 1991, disclose substituted imidazole, pyrrole, pyrazole, triazole and tetrazole compounds useful as angiotensin II receptor antagonists

### SUMMARY OF THE INVENTION

The present invention provides the use of an angiotensin II receptor antagonist in the manufacture of a medicament for the treatment of left ventricular hypertrophy.

### DESCRIPTION OF THE INVENTION

The present invention provides for the use of (E)-3-[2-n-butyl-1-{(4-carboxyphenyl)methyl}-1H-imidazol-5-yl]-2-(2-thienyl)methyl-2-propenoic acid, or a pharmaceutically acceptable salt thereof, in the manufacture of a medicament for the treatment of left ventricular hypertrophy.

A preferred salt is (E)-3-[2-n-butyl-1-{(4-carboxyphenyl)methyl}-1H-irnidazolyl-5-yl]-2-(2-thienyl)methyl-2-propenoic acid methanesulfonate.

The compounds for use in the invention are prepared following the methods described in European Patent Publication Number EP 0 403 159, published on December 19, 1990.

Angiotensin II antagonist activity is assessed by in vitro methods. In vitro antagonist activity is determined by the ability of the compounds to compete with ¹²⁵I-angiotensin II for binding to vascular angiotensin II receptors and by their ability to antagonize the contractile response to angiotensin II in the isolated rabbit aorta.

### Binding

The radioligand binding assay is a modification of a method previously described in detail (Gunther *et al*., Circ. Res. 47:278, 1980). A particular fraction from rat mesenteric arteries is incubated in Tris buffer with 80 pM of ¹²⁵I-angiotensin II with or without angiotensin II antagonists for 1 hour at 25°C. The incubation is terminated by rapid filtration and receptor bound ¹²⁵I-angiotensin II trapped on the filter is quantitated with a gamma counter. The potency of angiotensin II antagonists is expressed as the IC₅₀ which is the concentration of antagonist needed to displace 50% of the total specifically bound angiotensin II.

### Aorta

The ability of the compounds to antagonize angiotensin II induced vasoconstriction is examined in the rabbit aorta. Ring segments are cut from the rabbit thoracic aorta and suspended in organ baths containing physiological salt solution. The ring segments are mounted over metal supports and attached to force displacement transducers which are connected to a recorder. Cumulative concentration response curves to angiotensin II are performed in the absence of antagonist or following a 30-minute incubation with antagonist. Antagonist dissociation constants (K_{B}) are calculated by the dose ratio method using the mean effective concentrations.

In the therapeutic use for the treatment of left ventricular hypertrophy the AII receptor antagonizing compounds of this invention are incorporated into standard pharmaceutical compositions. They can be administered orally, parenterally, rectally, topically or transdermally.

The compounds of the instant invention and their pharmaceutically acceptable salts which are active when given orally can be formulated as liquids, for example syrups, suspensions or emulsions, tablets, capsules and lozenges.

A liquid formulation will generally consist of a suspension or solution of the compound or pharmaceutically acceptable salt in a suitable liquid carrier(s) for example, ethanol, glycerine, non-aqueous solvent, for example, polyethylene glycol, oils, or water with a suspending agent, preservative, flavouring or colouring agent.

A composition in the form of a tablet can be prepared using any suitable pharmaceutical carrier(s) routinely used for preparing solid formulations. Examples of such carriers include magnesium stearate, starch, lactose, sucrose and cellulose.

A composition in the form of a capsule can be prepared using routine encapsulation procedures. For example, pellets containing the active ingredient can be prepared using standard carriers and then filled into a hard gelatin capsule; alternatively, a dispersion or suspension can be prepared using any suitable pharmaceutical carrier(s), for example aqueous gums, celluloses, silicates or oils and the dispersion or suspension then filled into a soft gelatin capsule.

The compounds of the instant invention and their pharmaceutically acceptable salts which are active when administered parenterally (i.e. by injection or infusion) can be formulated as solutions or suspensions.

A composition for parenteral administration will generally consist of a solution or suspension of the active ingredient in a sterile aqueous carrier or parenterally acceptable oil, for example polyethylene glycol, polyvinyl pyrrolidone, lecithin, arachis oil or sesame oil. Alternatively, the solution can be lyophilised and then reconstituted with a suitable solvent just prior to administration.

A typical suppository composition comprises a compound of the instant invention or a pharmaceutically acceptable salt thereof which is active when administered in this way, with a binding and/or lubricating agent such as polymeric glycols, gelatins or cocoa butter or other low melting vegetable or synthetic waxes or fats.

A typical transdermal formulation comprises a conventional aqueous or non-aqueous vehicle, for example, a cream, ointment lotion or paste or in the form of a medicated plaster, patch or membrane.

For topical administration, the pharmaceutical compositions adapted include solutions, suspensions, ointments, and solid inserts. Typical pharmaceutically acceptable carriers are, for example, water, mixtures of water and water-miscible solvents such as lower alkanols or vegetable oils, and water soluble ophthalmologically acceptable non-toxic polymers, for example, cellulose derivatives such as methyl cellulose. The pharmaceutical preparation may also contain non-toxic auxiliary substances such as emulsifying, preserving, wetting, and bodying agents, as for example, polyethylene glycols; antibacterial components such as quaternary ammonium compounds; buffering ingredients such as alkali metal chloride; antioxidants such as sodium metabisulfite; and other conventional ingredients such as sorbitan monolaurate.

Preferably the composition is in unit dose form. Doses of the compounds of the instant invention in a pharmaceutical dosage unit will be an efficacious, non-toxic quantity selected from the range of 0.01 - 200 mg/kg of active compound, preferably 0.1 - 100 mg/kg. The selected dose is administered to a human patient in need of treatment of left ventricular hypertrophy induced by angiotensin II from 1-6 times daily, orally, rectally, topically, by injection, or continuously by infusion. Oral dosage units for human administration preferably contain from 10 to 500 mg of active compound. Lower dosages are used generally for parenteral administration. Oral administration is used when safe, effective, and convenient for the patient.

No unacceptable toxicological effects are expected when compounds of the invention are administered in accordance with the present invention.

The following examples are intended to illustrate the present invention. These are directed to pharmaceutical compositions produced according to this invention. These disclosed compositions include (E)-3-[2-n-butyl-1-{(4-carboxyphenyl)methyl}-1H-imidazol-5-yl]-2-(2-thienyl)methyl-2-propenoic acid, but therapeutically effective amounts of pharmaceutically acceptable salts as discussed hereinabove may be substituted.

### Example 1

An oral dosage form for administering orally active compounds of the invention is produced by screening, mixing and filling into hard gelatin capsules the ingredients in proportions, for example, as shown below.

| Ingredients | Amounts |
|---|---|
| (E)-3-[2-n-butyl-1-{(4-carboxyphenyl)methyl}-1H- | 100 mg |
| imidazol-5-yl]-2-(2-thienyl)methyl-2-propenoic acid magnesium stearate | 10 mg |
| lactose | 100 mg |

### Example 2

(E)-3-[2-n-Butyl-1-{(4-carboxyphenyl)methyl}-1H-imidazol-5-yl]-2-(2-thienyl)methyl-2-propenoic acid, 50 mg, is dispersed in 25 ml of normal saline to prepare an injectable preparation.

## Claims

1. The use of (E)-3-[2-n-butyl-1-{(4-carboxyphenyl)methyl}-1H-imidazol-5-yl]-2-(2-thienyl)methyl-2-propenoic acid, or a pharmaceutically acceptable salt thereof, in the manufacture of a medicament for the treatment of left ventricular hypertrophy.

2. The use of (E)-3-[2-n-butyl-1-{(4-carboxyphenyl)methyl}-1H-imidazol-5-yl]-2-(2-thienyl)methyl-2-propenoic acid methanesulfonate in the manufacture of a medicament for the treatment of left ventricular hypertrophy.

## Patentansprüche

1. Verwendung von (E)-3-[2-n-Butyl-1-{(4-carboxyphenyl)methyl}-1H-imidazol-5-yl]-2-(2-thienyl)methyl-2-propensäure oder einem pharmazeutisch verträglichen Salz davon für die Herstellung eines Medikaments zur Behandlung von Linksherzhypertrophie.

2. Verwendung von (E)-3-[2-n-Butyl-1-{(4-carboxyphenyl)methyl}-1H-imidazol-5-yl]-2-(2-thienyl)methyl-2-propensäuremethansulphonat für die Herstellung eines Medikaments zur Behandlung von Linksherzhypertrophie.

## Revendications

1. Utilisation d'acide (E)-3-[2-n-butyl-1-{(4-carboxyphényl)méthyl}-1H-imidazol-5-yl]-2-(2-thiényl)méthyl-2-propénoïque, ou d'un de ses sels pharmaceutiquement acceptables, dans la production d'un médicament destiné au traitement de l'hypertrophie ventriculaire gauche.

2. Utilisation de méthane-sulfonate d'acide (E)-3-[2-n-butyl-1-{(4-carboxyphényl)méthyl}-1H-imidazol-5-yl]-2-(2-thiényl)méthyl-2-propénoïque dans la production d'un médicament destiné au traitement de l'hypertrophie ventriculaire gauche.
